(19) Europäisches Patentamt
European Patent Office
Office européen des brevets

(11) Veröffentlichungsnummer: **0 457 204 B1**

(12) **EUROPÄISCHE PATENTSCHRIFT**

(45) Veröffentlichungstag der Patentschrift: **07.12.94**

(51) Int. Cl.5: **C07D 261/08**, A01N 43/72, C07D 261/18

(21) Anmeldenummer: **91107586.9**

(22) Anmeldetag: **10.05.91**

Die Akte enthält technische Angaben, die nach dem Eingang der Anmeldung eingereicht wurden und die nicht in dieser Patentschrift enthalten sind.

(54) **3-Isoxazolylbenzylester, ihre Herstellung und ihre Verwendung.**

(30) Priorität: **18.05.90 DE 4016049**

(43) Veröffentlichungstag der Anmeldung:
**21.11.91 Patentblatt 91/47**

(45) Bekanntmachung des Hinweises auf die Patenterteilung:
**07.12.94 Patentblatt 94/49**

(84) Benannte Vertragsstaaten:
**AT BE CH DE ES FR GB IT LI NL**

(56) Entgegenhaltungen:
**EP-A- 0 110 769**
**FR-A- 2 197 882**
**US-A- 4 153 707**

**JOURNAL OF AGRICULTURAL AND FOOD CHEMISTRY, Band 32, Nr. 5, September-Oktober1984, Seiten 1116-1121, Washington, DC; US; E.L. PLUMMER et al.: "Heterocyclicanalogues of substituted [1,1'-biphenyl]-3-methylpyrethroid"**

(73) Patentinhaber: **BASF Aktiengesellschaft**
**Carl-Bosch-Strasse 38**
**D-67063 Ludwigshafen (DE)**

(72) Erfinder: **Mueller, Stefan, Dr.**
**Closweg 7**
**W-6720 Speyer (DE)**
Erfinder: **Theobald, Hans, Dr.**
**Oueichstrasse 6**
**W-6703 Limburgerhof (DE)**
Erfinder: **Wolf, Bernd, Dr.**
**Hallbergstrasse 4**
**W-6701 Fussgoenheim (DE)**
Erfinder: **Hofmeister, Peter, Dr.**
**Bernard-Humblot-Strasse 12**
**W-6730 Neustadt (DE)**
Erfinder: **Kardorff, Uwe, Dr.**
**D 3,4**
**W-6800 Mannheim 1 (DE)**
Erfinder: **Kuenast, Christoph, Dr.**
**Salierstrasse 2**
**W-6701 Otterstadt (DE)**

**EP 0 457 204 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 3-Isoxazolylbenzylester der allgemeinen Formeln Ia und Ib

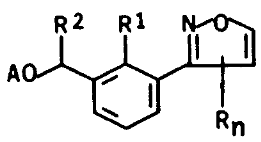

Ia

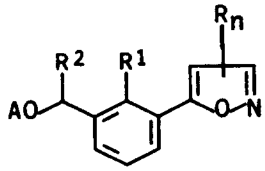

Ib

in denen die Substituenten und der Index folgende Bedeutung haben:

| | |
|---|---|
| R | Halogen; $C_1$-$C_4$-Alkyl; $C_1$-$C_4$-Halogenalkyl; $C_1$-$C_4$-Alkoxy; $C_1$-$C_4$-Halogenalkoxy; $C_2$-$C_4$-Alkenyl; $C_2$-$C_4$-Halogenalkenyl; Phenylethenyl, welches ein bis fünf Halogenatome tragen kann; $C_2$-$C_4$-Alkinyl; $C_3$-$C_8$-Cycloalkyl; Phenyl, Naphthyl; 5- oder 6-gliedriges Hetaryl; $CO_2R^3$ oder $CONR^4R^5$, wobei |
| $R^3$, $R^4$ und $R^5$ | Wasserstoff oder $C_1$-$C_6$-Alkyl bedeuten; |
| n | 0, 1 oder 2, wobei die Reste R verschieden sein können, wenn n 2 bedeutet; |
| $R^1$ | Halogen oder $C_1$-$C_4$-Alkyl; |
| $R^2$ | Wasserstoff; $C_1$-$C_4$-Alkyl; $C_2$-$C_4$-Alkenyl; $C_2$-$C_4$-Alkinyl oder Cyano; |
| A | der Carbonylrest einer Pyrethroidsäure der allgemeinen Formeln IIa oder IIb |

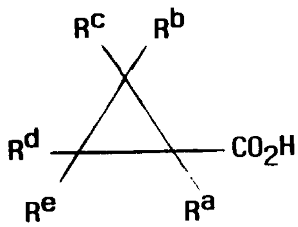

IIa

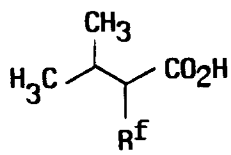

IIb

,

in denen die Substituenten folgende Bedeutung haben:

| | |
|---|---|
| $R^a$ | Wasserstoff, $C_1$-$C_4$-Alkyl oder Phenyl, wobei der aromatische Ring ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio; |
| $R^b$, $R^c$, $R^d$ | Wasserstoff; Halogen oder $C_1$-$C_4$-Alkyl; |
| $R^e$ | Wasserstoff; Halogen; $C_1$-$C_4$-Alkyl; $C_1$-$C_4$-Halogenalkyl; $C_2$-$C_6$-Alkenyl oder $C_2$-$C_6$-Alkdienyl, welche ein bis acht Halogenatome und/oder einen der folgenden Reste tragen können: $C_1$-$C_4$-Alkoxycarbonyl oder Phenyl, wobei der Phenylrest seinerseits ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio; $C_3$-$C_7$-Cycloalkylidenmethyl oder |
| $R^d$ und $R^e$ | gemeinsam eine 2,2H-Indenspirogruppe; |
| $R^f$ | ein ein- oder zweikerniges aromatisches oder heteroaromatisches Ringsystem, welches als Heteroatom ein Stickstoffatom enthalten kann, wobei dieses Ringsystem ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio oder<br>eine Phenylaminogruppe, wobei der Phenylring seinerseits ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio. |

Des weiteren betrifft die Erfindung 3-Isoxazolylbenzylderivate der allgemeinen Formeln IIIa und IIIb

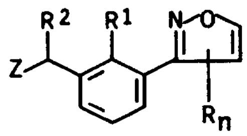

IIIa

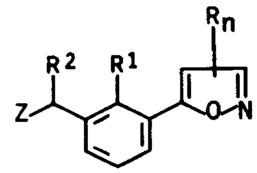

IIIb

in der die Substituenten R, $R^1$ und $R^2$ und der Index n die vorstehend gegebene Bedeutung haben und Z für Hydroxy und Halogen steht.

Es ist bekannt, daß bestimmte Benzylester mit metaständigem Heteroaromaten (z.B. Pyrrolylbenzylester, 3-Furanylbenzylester, 3-Thienylbenzylester) insektizid bzw. akarizid wirksam sind (PCT Int. Appl. WO 82/1368; US-Patent 4 426 524; J. Agric. Food Chem. 32, 1116 (1984); Eur. Pat. Appl. EP 110 769). Die insektizide bzw. akarizide Wirksamkeit dieser Ester läßt jedoch bei niedrigen Aufwandmengen zu wünschen übrig.

Der Erfindung lagen daher neue 3-Hetarylbenzylester mit verbesserten Eigenschaften hinsichtlich der biologischen Aktivität als Aufgabe zugrunde.

Demgemäß würden die eingangs definierten 3-Isoxazolyl-benzylester Ia und Ib gefunden.

Außerdem wurden die neuen Zwischenprodukte IIIa und IIIb, Verfahren zur Herstellung der Verbindungen Ia, Ib, IIIa und IIIb sowie die Verwendung der Verbindungen Ia und Ib zur Bekämpfung von Schädlingen gefunden.

Man erhält die Verbindungen Ia und Ib dadurch, daß man einen 3-Isoxazolyl-benzylalkohol oder ein entsprechendes Benzylhalogenid der Formel IIIa oder IIIb in an sich bekannter Weise mit einer bei Pyrethroiden üblichen Carbonsäure A-OH oder deren Derivaten umsetzt.

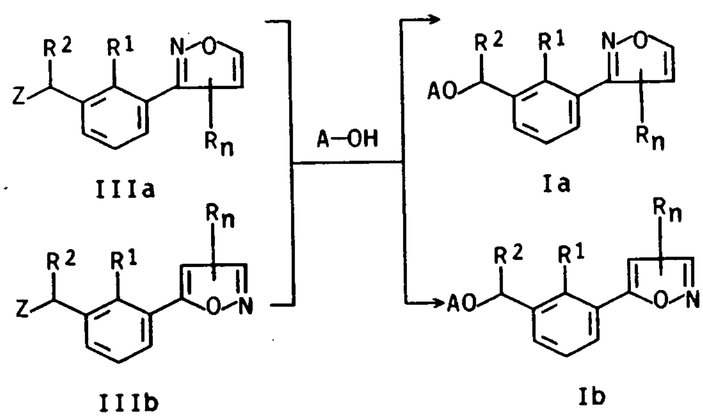


IIIa

Ia

IIIb

Ib

Geeignete Reste Z sind Hydroxy oder Halogen wie insbesondere Chlor und Brom. Anstelle der Pyrethroidsäure können auch die aktivierten Säurederivate wie Anhydride, Halogenide, z.B. Chloride und Bromide, oder Imidazolide Anwendung finden.

Als Lösungsmittel für diese Umsetzung dienen organische Lösungsmittel wie aliphatische und aromatische, gegebenenfalls chlorierte Kohlenwasserstoffe, z.B. Petrolether, Benzol, Toluol, Xylol, Benzin, Dichlormethan, Chloroform, Tetrachlormethan, 1,2-Dichlorethan, Chlorbenzol; Ether wie Diethyl- und Di-n-butylether, Methyl-tert.-butylether, Tetrahydrofuran, Dioxan; Ketone, beispielsweise Aceton, Methylethylketon, Methylisopropylketon; ferner Nitrile wie Acetonitril und Propionitril sowie entsprechende Gemische.

Je nachdem, nach welchem der allgemein beKannten Verfahren die Veresterung durchgeführt wird, Kann der Zusatz von Basen oder Reaktionsbeschleunigern oder die Verwendung der dafür üblichen Basen als Lösungsmittel empfehlenswert sein (vgl. Houben-Weyl, Methoden der organ. Chemie, Band VIII, S. 541 ff, Georg-Thieme-Verlag, Stuttgart 1952).

Als Säurebindemittel eignen sich die üblichen basischen Mittel, insbesondere aliphatische, aromatische und heterocyclische Amine, z.B. Triethylamin, Dimethylamin, Piperidin, Dimethylanilin, Dimethylbenzylamin, Pyridin und 2-Picolin.

Die Base wird im allgemeinen in einem Überschuß bis zu 5,0 mol.-Äq., vorzugsweise bis zu 2,0 mol.-Äq., insbesondere von 1,1 mol.-Äq. bis 1,35 mol.-Äq. bezogen auf das Halogenid der Pyrethroidsäure bzw. das Benzylhalogenid verwendet.

Die Umsetzung verläuft gewöhnlich oberhalb von 0 °C mit ausreichender Geschwindigkeit. Da sie meist unter Wärmeentwicklung verläuft, kann es von Vorteil sein, eine Kühlmöglichkeit vorzusehen.

In der Regel erfolgt die Umsetzung bei Temperaturen von -40 °C bis 140 °C, vorzugsweise 0 °C bis 100 °C, insbesondere 10 °C bis 50 °C.

Die Umsetzung kann in an sich bekannter Weise durch Zusatz von Katalysatoren wie Schwefelsäure, Halogenwasserstoff, Sulfonsäuren und sauren Ionenaustauschern beschleunigt und das Veresterungsgleichgewicht im gewünschten Sinne verschoben werden, indem man dem Reaktionsgemisch das Wasser oder den Ester I entzieht, z.B. durch azeotrope Destillation oder durch Bindung des Wassers in Schwefel- oder Halogenwasserstoffsäure.

Im allgemeinen werden die 3-Isoxazolylbenzylalkoholderivate IIIa bzw. IIIb und die Pyrethroidsäuren A-OH bzw. deren Derivate in äquimolaren Mengen miteinander umgesetzt. Es Kann vorteilhaft für die Ausbeute sein, das Benzylderivat in einem über- oder Unterschuß bezogen auf die Säure A-OH oder ihr Derivat einzusetzen.

In einigen Fällen ist es sinnvoll und vorteilhaft, die Verbindungen der Formel IIIa und IIIb, insbesondere dann, wenn $R^2$ in der allgemeinen Formel IIIa und IIIb die Bedeutung einer Cyanogruppe hat, in situ zu verestern.

Die erfindungsgemäßen Ester können außerdem noch nach praktisch allen bekannten Verfahren der Estersynthese hergestellt werden, beispielsweise durch Umsetzung von entsprechenden Säureanhydriden mit den Alkoholen der Formel IIIa und IIIb, durch Umsetzung von entsprechenden Salzen mit Derivaten der Alkohole der Formel IIIa und IIIb, wie z. B. den entsprechenden Benzylbromiden oder Benzylchloriden, oder durch Umesterung (vgl. Houben-Weyl a.a.O. S. 508-628).

Es versteht sich, daß die Verbindungen der Formel Ia und Ib in jedem Falle in Form von einheitlichen Enantiomeren bzw. Diastereomeren, in vielen Fällen auch in Form von Gemischen der Strukturisomeren vorkommen und als Wirkstoffe angewandt werden können, die je nach den Ausgangsstoffen und den Umsetzungsbedingungen in einheitlicher Form oder als Gemische auftreten. Die Gemische können in üblicher Weise in ihre sterisch einheitlichen Bestandteile aufgetrennt werden; ihre biologische Wirkung ist in Einzelfällen von ihrer sterischen Konfiguration abhängig.

Die eingesetzten Pyrethroidsäuren und ihre Derivate sind beispielsweise in Wegler, Chemie der Pflanzenschutz- und Schädlingsbekämpfungsmittel, Band 7, Springer Verlag, Berlin, Heidelberg, New York 1981 beschrieben.

Die für die Herstellung der Verbindungen Ia und Ib benötigten 3-Isoxazolyl-benzylderivate IIIa und IIIb sind auf verschiedenen Wegen zugänglich.

So erhält man die Verbindungen IIIa dadurch, daß man einen geschützten 3-Formylbenzylalkohol der allgemeinen Formel IV in an sich bekannter Weise in einem inerten organischen Lösungsmittel im allgemeinen in Gegenwart einer Base in das entsprechende Oxim Va überführt, Va anschließend in einem inerten organischen Lösungsmittel in Gegenwart eines Oxidationsmittels und einer Base an ein Alkin der Formel VIa addiert und aus dem so erhaltenen 3-Isoxazolyl-benzylether VIIa in an sich bekanntere Weise in einem inerten organischen Lösungsmittel in Gegenwart einer Säure oder eines Katalysators die Schutzgruppe abspaltet.

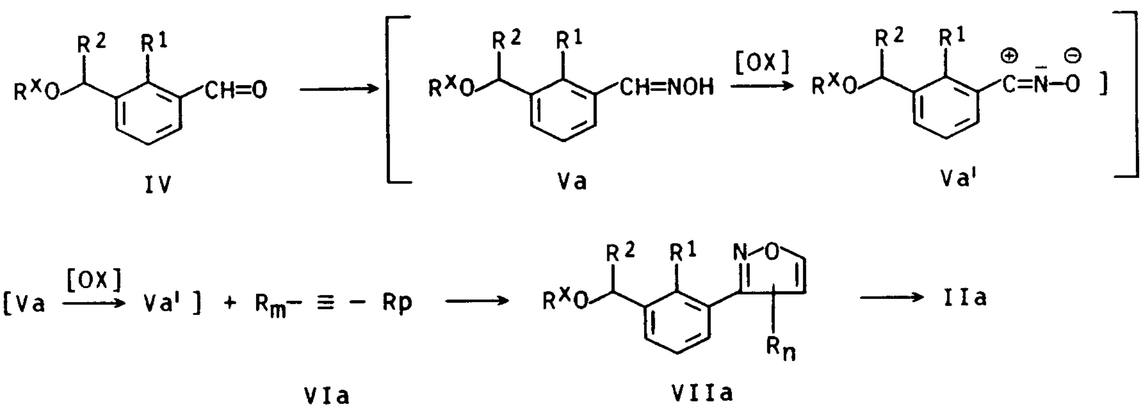

IV Va Va'

VIa VIIa IIa

In den Formeln IV, Va, Va' und VIIa bedeutet $R^x$ eine Schutzgruppe wie Methoxymethyl, 2-Methoxyethoxymethyl, Tetrahydro-2-pyranyl, Tetrahydro-2-furanyl, tert.-Butyl-dimethylsilyl und Trimethylsilyl.

m und p in der Formel VIa stehen für 0 und 1, wobei die Summe m + p dem Wert von n entspricht.

Die Umsetzung des Aldehyds IV zum Oxim Va erfolgt in an sich bekannter Weise (Houben-Weyl, Methoden der organischen Chemie, Bd. VII/1, S. 471 f, Bd. X/4, S. 56 f).

Die anschließende Spaltung des 3-Isoxazolylbenzylethers VIIa zum 3-Isoxazolyl-benzylalkohol erfolgt in an sich bekannter Weise (T. Greene, Protective Groups in Organic Chemistry, J. Wiley & Sons, New York 1981; Tietze et al., Reaktionen und Synthesen, Georg-Thieme-Verlag 1981, S. 363 f.) in einem inerten organischen Lösungsmittel in Gegenwart einer Säure oder eines Katalysators.

Die 3-Isoxazolyl-benzylalkohole IIIb erhält man in ähnlicher Weise dadurch, daß man einen ethergeschützten 3-Formylbenzylalkohol der allgemeinen Formel IV in an sich bekannter Weise in einem inerten organischen Lösungsmittel in Gegenwart einer Base mittels eines Phosphonium- oder Phosphonat-Reagens' in einer Wittig bzw. Horner-Wittig Reaktion in das entsprechende 3-Bromvinylderivat VIII überführt, VIII anschließend in einem inerten organischen Lösungsmittel in Gegenwart einer Base in ein Alkin der Formel VIb überführt, welches danach in an sich bekannter Weise in einem inerten organischen Lösungsmittel in Gegenwart eines Oxidationsmittels und einer Base an ein Oxim der Formel Vb zum 3-Isoxazolylbenzylether VIIb addiert wird, aus dem in an sich bekannter Weise die Schutzgruppe abgespalten wird.

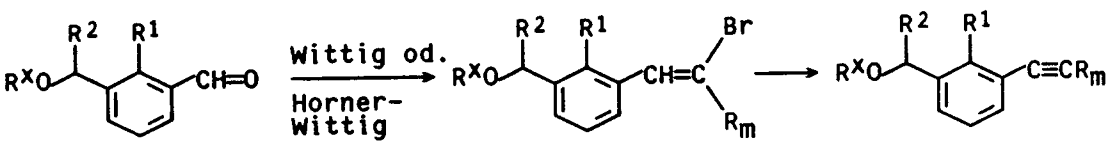


IV VIII VIb

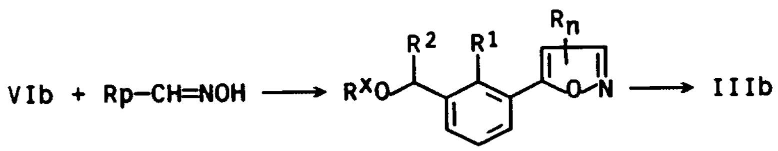


Vb VIIb

Bevorzugte Wittig bzw. Horner-Wittig Reagentien sind die Triphenylphosphoniumhalogenide und die Diethylphosphonate.

Die Reste $R^x$ (Formeln IV, IX, VIb und VIIb), $R_m$ (Formeln VIII, IX und VIb) und $R_p$ (Formel Vb) haben die vorstehend gegebene Bedeutung.

Die Wittig oder Horner-Wittig Umsetzung des Aldehyds IV erfolgt in an sich bekannter Weise (z.B. Liebigs Ann. Chem., 1980, S. 2061 f; Synthesis 1975, S. 458 f.; DE-A 3 927 479).

Die Umsetzung von Vb mit VIb und die anschließende Spaltung des Ethers VIIb erfolgt analog den vorstehend für die Umsetzung von Va mit VIa und die Spaltung des Ethers VIIa beschriebenen Bedingungen.

Für die Herstellung der 3-Isoxazolyl-benzylalkoholen IIIb, in denen n 0 oder 1 bedeutet, kommen insbesondere auch folgende aus der Literatur bekannte Umsetzungen in Betracht:

1. Analog zu Tietze et al, Reaktionen und Synthesen, Georg-Thieme-Verlag 1981, S. 299 f

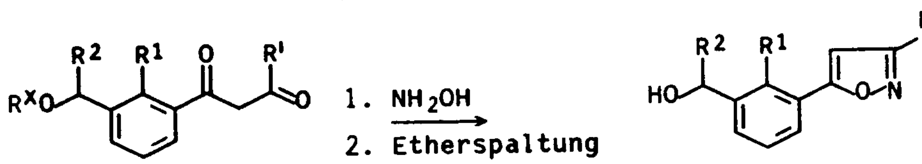


IIIb

(R' = Wasserstoff oder ein Rest R)

2. Analog zu Huisgen et al., Chem. Ber. 1973, S. 3291 f.

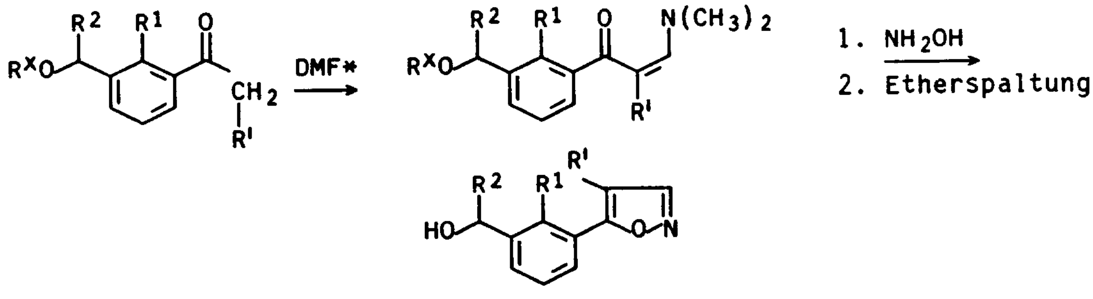


IIIb

* DMF = Dimethylformamid

3. Analog zu Bowden et al., J. Chem. Soc. 1946, 953 f.

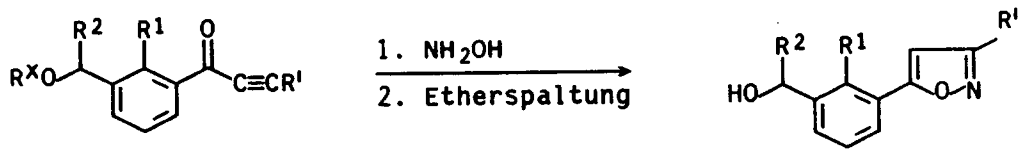


Die Herstellung des für die Synthese der 3-Isoxazolyl-benzylalkohole IIIa und IIIb benötigten 3-Formylbenzylethers IV erfolgt nach an sich bekannten Methoden (DE-A 3 927 479) gemäß dem folgenden Reaktionsschema:

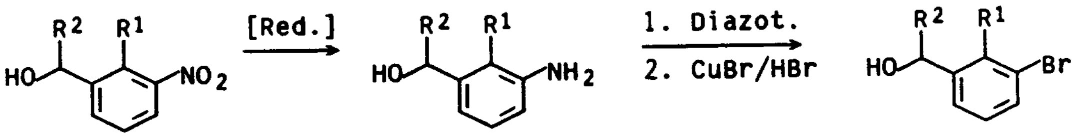


X XI XII

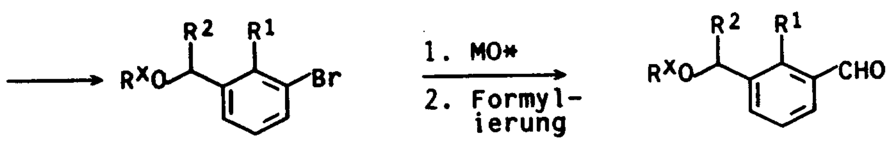


XIII IV

* MO = Metall oder Metallorganyl

Die Reduktion von X zu XI und die Diazotierung von XI zu XII Kann nach den in EP-A 54 180 beschriebenen Verfahren erfolgen.

Der Alkohol XII läßt sich nach der in Tietze/Eicher (Reaktionen und Synthesen, Thieme Verlag, 1981, Seite 184) beschriebenen Methode in den Ether XIII umwandeln.

Zur Herstellung des Aldeyhds IV Kommt die Umsetzung der entsprechenden metallorganischen Verbindungen (Grignardverbindung oder Lithiumorganylverbindung) mit bestimmten Formamiden wie z.B. Dimethylformamid, 1-Formylpiperidin oder 2-(Formyl-methylamino)-pyridin in Frage (vgl. Houben-Weyl, Methoden der organischen Chemie, Band E 3, S. 130).

Die Benzylalkohole der allgemeinen Formel III, in der $R^2$ Cyano, $C_2$-$C_4$-Alkinyl, $C_2$-$C_4$-Alkenyl oder $C_1$-$C_4$-Alkyl bedeutet, erhält man vorteilhaft dadurch, daß man zunächst die unsubstituierten Benzylalkohole mit $R^2$ = H zu den entsprechenden Benzaldehyden IX oxidiert.

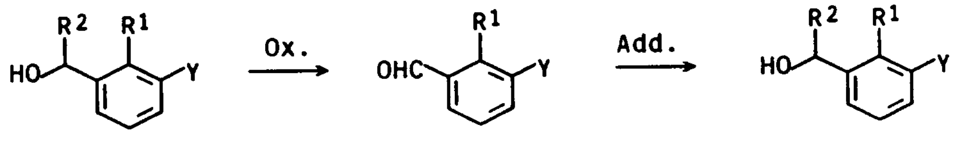


III ($R^2$ = H) IX III $R^2 \neq$ H

Z in den Formeln III und XIV bedeutet hier den Isoxazoylrest. Als Oxidationsmittel kommen alle gängigen Oxidationsmittel, die primäre Alkohole in Aldehyde überführen, in Frage (Houben-Weyl, Methoden der organischen Chemie, Band E3, S. 265 ff). Besonders geeignet sind Verbindungen mit Übergangsmetallen in höherer Oxidationsstufe, z.B. Pyridiniumchlorochromat.

Die Benzaldehyde IX können in einem anschließenden Reaktionsschritt in an sich bekannter Weise zu den substituierten Benzylalkoholen mit $R^2 \neq H$ umgesetzt werden. Dabei wird

a) im Fall von $R^2$ = CN der Benzaldehyd mit Blausäure oder einem Metallcyanid ggf. in Gegenwart einer Säure umgesetzt;

b) im Fall von $R^2$ = $C_2$-$C_4$-Alkinyl, $C_2$-$C_4$-Alkenyl oder $C_1$-$C_4$-Alkyl, der Benzaldehyd mit Metallorganylen $MR^2$ oder $R^2MHal$, wobei M für Alkali-, Erdalkali- oder Übergangsmetalle und Hal für Halogen steht, zur Reaktion gebracht.

Zur Herstellung der Cyanhydrine werden die Benzaldehyde mit Blausäure, in situ aus Metallcyaniden erzeugter Blausäure oder Metallcyaniden in Gegenwart von Alkalihydrogensulfitlösung umgesetzt, wobei gegebenenfalls basische Katalysatoren wie Kaliumcarbonat oder Phasentransferkatalysatoren wie z.B. Benzyltriethylammoniumchlorid zugegeben werden.

Als Metallcyanide werden bevorzugt Alkalimetallcyanide wie z.B. Natrium- oder Kaliumcyanid verwendet.

Die Umsetzung erfolgt in an sich bekannter Weise, z.B. wie in Houben-Weyl, Methoden der org. Chemie, Band VIII, S. 274-278, Ausgabe 1952 und Band E5, S. 1413 ff, 1985, beschrieben.

Als Metallorganyle eignen sich die entsprechenden metallorganischen Verbindungen, insbesondere Lithiumorganylverbindungen $LiR^2$ wie Methyllithium, Ethyllithium, Butyllithium oder die entsprechenden Grignardverbindungen $R^2MgHal$, worin Hal für Chlor, Brom oder Jod steht, z.B. Methylmagnesiumbromid, Ethylmagnesiumchlorid, Propylmagnesiumiodid oder Vinylmagnesiumiodid.

Die Umsetzung mit Metallorganylen kann in an sich bekannter Weise, z.B. wie in Houben-Weyl, Methoden der org. Chemie, Band 13/2a, S. 285ff, 1973 beschrieben in einem inerten organischen Lösungsmittel wie Ether oder Tetrahydrofuran unter Schutzgas durchgeführt werden, so daß sich nähere Ausführungen hierzu erübrigen.

Im Hinblick auf die bestimmungsgemäße Verwendung der Verbindungen Ia und Ib Kommen als Substituenten beispielsweise folgende Reste in Betracht:

R Halogen wie Fluor, Chlor, Brom und Jod;

Alkyl wie Methyl, Ethyl, Propyl, 1-Methylethyl, Butyl, 1-Methylpropyl, 2-Methylpropyl und 1,1-Dimethylethyl, vorzugsweise Methyl und Ethyl;

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;

Alkoxy wie Methoxy, Ethoxy, Propoxy, 1-Methylethoxy, Butoxy, 1-Methylpropoxy, 2-Methylpropoxy und 1,1-Dimethylethoxy;

Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy;

Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl;

Halogenalkenyl wie 2,2-Dichlorethenyl, 2,2-Dibromethenyl, 2,2-Difluorethenyl, 2-Chlor-2-fluorethenyl, 2-Brom-2-chlorethenyl, 2-Brom-2-fluorethenyl, 2,2-Di(trifluormethyl)-ethenyl, 2-Chlor-2-trifluormethylethenyl und 2-Fluor-2-trifluormethyl-ethenyl; Phenylethenyl, welches ein bis fünf Halogenatome wie insbesondere Fluor und Chlor sowohl am Phenylring als auch an der Ethenylgruppierung tragen kann, insbesondere 2-Chlor-2-(4-chlorphenyl)ethenyl;

Alkinyl wie Ethinyl, 1-Propinyl, 2-Propinyl, 1-Butinyl, 2-Butinyl, 3-Butinyl, 1-Methyl-2-propinyl;
Cycloalkyl wie Cyclopropyl, Cyclobutyl, Cyclopentyl, Cyclohexyl und Cycloheptyl;
Phenyl und Naphthyl;
5- oder 6-gliedriges Hetaryl, z.B. Pyrrolyl, Pyrazolyl, Imidazolyl, Isoxazolyl, Oxazolyl, Isothiazolyl, Thiazolyl, Furanyl, Thienyl und Pyridyl;
eine Carboxylatgruppe $CO_2R^3$ oder eine Carboxamidgruppe $CONR^4R^5$, wobei

$R^3$, $R^4$ und $R^5$ für Wasserstoff oder Alkyl wie vorstehend genannt sowie für Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl stehen;

$R^3$ vorzugsweise Methyl oder Ethyl;

$R^4$ vorzugsweise Methyl oder Ethyl;

$R^5$ vorzugsweise Methyl oder Ethyl;

n 0, 1 oder 2, wobei die Reste R verschieden sein können, wenn n 2 bedeutet;

$R^1$ Halogen wie bei R genannt, vorzugsweise Fluor und Chlor;
Alkyl wie bei $R^4$ genannt, vorzugsweise Methyl und Ethyl;

$R^2$ Wasserstoff;
Alkyl wie bei R genannt;
Alkenyl wie bei R genannt;
Alkinyl wie bei R genannt, vorzugsweise Ethinyl;
oder Cyano.

Als Carbonylrest A eignen sich die Reste der Formel II.A und II.B

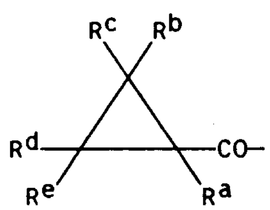

II.A

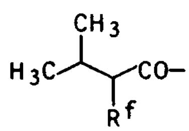

II.B

in denen die Substituenten folgende Bedeutung haben:

$R^a$ Wasserstoff;
Alkyl wie bei R genannt oder
Phenyl, welches ein bis fünf Halogenatome wie bei R genannt, vorzugsweise Chlor, insbesondere in 4-Position und/oder ein bis drei der folgenden Reste tragen kann: Alkyl wie bei R genannt, Halogenalkyl wie bei R genannt, Alkoxy wie bei R genannt, vorzugsweise Ethoxy, insbesondere in 4-Position; Halogenalkoxy wie bei R genannt, oder
Alkylthio wie Methylthio, Ethylthio, Propylthio, 1-Methylethylthio, Butylthio, 1-Methylpropylthio, 2-Methylpropylthio und 1,1-Dimethylethylthio;

$R^b$, $R^c$ und $R^d$ unabhängig voneinander Wasserstoff; Halogen wie bei R genannt, vorzugsweise Chlor und Brom, oder
Alkyl wie bei R genannt, vorzugsweise Methyl;

$R^b$ Halogen oder Alkyl wie bei R genannt, vorzugsweise Chlor, Brom oder Methyl;

$R^c$ im allgemeinen und im besonderen die bei $R^b$ genannten Reste;

$R^d$ insbesondere Wasserstoff oder Methyl;

$R^e$ Wasserstoff;
Halogen wie bei R genannt, vorzugseise Chlor oder Brom;
Alkyl wie bei R genannt, vorzugsweise Methyl;
Halogenalkyl wie bei R genannt; Alkenyl wie bei R genannt sowie
1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Mthyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, vorzugsweise Ethenyl, 1-Propenyl und 1-Methyl-1-propenyl, welches ein bis acht Halogenatome wie bei R genannt, vorzugsweise Fluor, Chlor und/oder Brom und/oder einen der folgenden Reste tragen kann:

Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl und 1,1-Dimethyl-ethoxycarbonyl, vorzugsweise Methoxycarbonyl oder Phenyl, welches ein bis fünf Halogenatome wie bei R genannt und/oder ein bis drei der folgenden Reste tragen kann: Alkyl wie bei R genannt, vorzugsweise tert.-Butyl; Halogenalkyl wie bei R genannt, Alkoxy wie bei R genannt, Halogenalkoxy wie bei R genannt, oder

Alkylthio wie Methylthio, Ethylthio, Propylthio, 1-Methylethylthio, Butylthio, 1-Methylpropylthio, 2-Methylpropylthio und 1,1-Dimethylethylthio;

Cycloalkylidenmethyl wie Cyclopropylidenmethyl, Cyclobutylidenmethyl, Cyclopentylidenmethyl, Cyclohexylidenmethyl und Cycloheptylidenmethyl, vorzugsweise Cyclopentylidenmethyl;

$R^f$ ein ein- oder zweikerniges aromatisches oder heteroaromatisches Ringsystem, welches als Heteroatom ein Stickstoffatom enthalten kann;

Phenyl, welches ein bis fünf Halogenatome wie bei R genannt, vorzugsweise Fluor und Chlor, insbesondere in 4-Position und/oder ein bis drei der folgenden Reste tragen Kann: Alkyl wie bei R genannt, vorzugsweise 1,1-Dimethylethyl, insbesondere in 4-Position; Halogenalkyl wie bei R genannt, vorzugsweise Difluormethyl; Alkoxy wie bei R genannt, Halogenalkoxy wie bei R genannt, oder

Alkylthio wie Methylthio, Ethylthio, Propylthio, 1-Methylethylthio, Butylthio, 1-Methylpropylthio, 2-Methylpropylthio und 1,1-Dimethylethylthio oder eine Phenylaminogruppe, wobei der Phenylring seinerseits ein bis fünf Halogenatome wie bei R genannt, vorzugsweise Fluor und Chlor, insbesondere in 2-Position und/oder ein bis drei der folgenden Reste tragen kann: Alkyl wie bei R genannt, vorzugsweise Methyl, Halogenalkyl wie bei R genannt, vorzugsweise Trifluormethyl; Alkoxy wie bei R genannt, vorzugsweise Methoxy, Halogenalkoxy wie bei R genannt, oder

Alkylthio wie Methylthio, Ethylthio, Propylthio, 1-Methylethylthio, Butylthio, 1-Methylpropylthio, 2-Methylpropylthio und 1,1-Dimethylethylthio.

Besonders bevorzugt als Substituent A sind die Carbonylreste der Pyrethroidsäuren der Formeln IIa bzw. IIb, die in den nachfolgenden Tabellen wiedergegeben sind:

Pyrethroidsäuren der allgemeinen Formel IIa

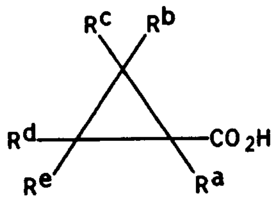

IIa

| Formel Nr. | $R^a$ | $R^b$ | $R^c$ | $R^d$ | $R^e$ |
|---|---|---|---|---|---|
| A.001 | H | $CH_3$ | $CH_3$ | H | $CH=C(CH_3)_2$ |
| A.002 | H | $CH_3$ | $CH_3$ | H | $CH=CCl_2$ |
| A.003 | H | $CH_3$ | $CH_3$ | H | $CH=CCl-CF_3$ |
| A.004 | H | $CH_3$ | $CH_3$ | H | $CH=CBr_2$ |
| A.005 | H | $CH_3$ | $CH_3$ | H | $CH=CF_2$ |
| A.006 | H | $CH_3$ | $CH_3$ | H | $CH=CF-CF_3$ |
| A.007 | H | $CH_3$ | $CH_3$ | H | $CH=C(CF_3)_2$ |
| A.008 | H | $CH_3$ | $CH_3$ | $CH_3$ | $CH_3$ |
| A.009 | $4-Cl-C_6H_4$ | H | H | H | H |
| A.010 | $4-OCH_2CH_3-C_6H_4$ | Cl | Cl | H | H |
| A.011 | H | $CH_3$ | $CH_3$ | H | $CH=CCl-(4-Cl-C_6H_4)$ |
| A.012 | H | $CH_3$ | $CH_3$ | H | $CH=CH-CH=CH_2$ |
| A.013 | H | $CH_3$ | $CH_3$ | H | $CH=C(CH_3)-CO_2CH_3$ |
| A.014 | H | $CH_3$ | $CH_3$ | 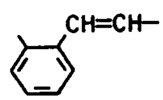 | * |
| A.015 | H | $CH_3$ | $CH_3$ | H | Cyclopentyliden-methyl |
| A.016 | H | $CH_3$ | $CH_3$ | H | $CHBr-CBrCl_2$ |
| A.017 | H | $CH_3$ | $CH_3$ | H | $4-(CH_3)_3C-C_6H_4$ |

* 2,2H-Indenspiro-Gruppe

Pyrethroidsäuren der allgemeinen Formel IIb

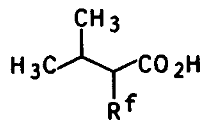

IIb

| Formel Nr. | $R^f$ |
|---|---|
| B.001 | $4-Cl-C_6H_4$ |
| B.002 | $4-F-C_6H_4$ |
| B.003 | $4-OCHF_2-C_6H_4$ |
| B.004 | $NH-(2-Cl,4-CF_3-C_6H_3)$ |
| B.005 | $NH-(2-F,4-CF_3-C_6H_3)$ |
| B.006 | $NH-(4-CF_3-C_6H_3)$ |
| B.007 | 1-Pyrrolyl |
| B.008 | $3-CH_3$-1-Pyrrolyl |
| B.009 | $3,4-(CH_3)_2$-1-pyrrolyl |
| B.010 | $2,5-(CH_3)_2$-1-pyrrolyl |
| B.011 | 2-Isoindolyl |

Bevorzugt im Hinblick auf ihre biologische Wirkung zur Schädlingsbekämpfung sind solche 3-Isoxazolyl-benzylester der allgemeinen Formeln Ia und Ib, in denen $R^1$ Methyl oder Ethyl und $R^2$ Wasserstoff bedeutet.

Außerdem sind 3-Isoxazolyl-benzylester der allgemeinen Formel Ia und Ib bevorzugt, in denen $R^1$ Methyl oder Ethyl und $R^2$ Wasserstoff bedeutet und n für 0 steht.

Insbesondere werden solche 3-Isoxazolylbenzylester Ia und Ib bevorzugt, in denen A für einen Carbonylrest einer Pyrethroidsäure der allgemeinen Formel IIa steht, in der

- $R^a$ und $R^d$ Wasserstoff bedeuten,
- $R^b$ und $R^c$ für Methyl und
- $R^e$ für $C_2$-$C_4$-Alkenyl, welches zwei bis sechs Halogenatome trägt,

steht.

Ganz besonders bevorzugt werden solche 3-Isoxazolylbenzylester Ia und Ib, in denen A der Carbonylrest der Pyrethroidsäure A.002, A.003 oder A.005 ist.

Beispiele für besonders aktive Verbindungen Ia und Ib sind in den folgenden Tabellen A und B wiedergegeben, wobei der Rest A durch die Formel Nr. der entsprechenden Säure charakterisiert wird.

Tabelle A

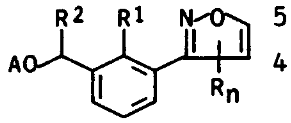 Ia

| A | $R^1$ | $R^2$ | $R_n$ |
|---|---|---|---|
| A.001 | $CH_3$ | H | - |
| A.002 | $CH_3$ | H | - |
| A.004 | $CH_3$ | H | - |
| A.005 | $CH_3$ | H | - |
| B.001 | $CH_3$ | H | - |
| B.005 | $CH_3$ | H | - |
| A.001 | $CH_3$ | CN | - |
| A.002 | $CH_3$ | CN | - |
| A.003 | $CH_3$ | CN | - |
| A.004 | $CH_3$ | CN | - |
| A.005 | $CH_3$ | CN | - |
| B.001 | $CH_3$ | CN | - |
| B.005 | $CH_3$ | CN | - |
| A.001 | $CH_3$ | C≡CH | - |
| A.002 | $CH_3$ | C≡CH | - |
| A.003 | $CH_3$ | C≡CH | - |
| A.004 | $CH_3$ | C≡CH | - |
| A.005 | $CH_3$ | C≡CH | - |
| B.001 | $CH_3$ | C≡CH | - |
| B.005 | $CH_3$ | C≡CH | - |
| A.001 | $CH_3$ | H | 5-$COOCH_3$ |
| A.002 | $CH_3$ | H | 5-$COOCH_3$ |
| A.003 | $CH_3$ | H | 5-$COOCH_3$ |
| A.004 | $CH_3$ | H | 5-$COOCH_3$ |
| A.005 | $CH_3$ | H | 5-$COOCH_3$ |
| B.001 | $CH_3$ | H | 5-$COOCH_3$ |

Tabelle A - Fortsetzung -

| A | $R^1$ | $R^2$ | $R_n$ |
|---|---|---|---|
| B.005 | $CH_3$ | H | 5–$COOCH_3$ |
| A.001 | $CH_3$ | CN | 5–$COOCH_3$ |
| A.002 | $CH_3$ | CN | 5–$COOCH_3$ |
| A.003 | $CH_3$ | CN | 5–$COOCH_3$ |
| A.004 | $CH_3$ | CN | 5–$COOCH_3$ |
| A.005 | $CH_3$ | CN | 5–$COOCH_3$ |
| B.001 | $CH_3$ | CN | 5–$COOCH_3$ |
| B.005 | $CH_3$ | CN | 5–$COOCH_3$ |
| A.001 | $CH_3$ | C≡CH | 5–$COOCH_3$ |
| B.002 | $CH_3$ | C≡CH | 5–$COOCH_3$ |
| A.003 | $CH_3$ | C≡CH | 5–$COOCH_3$ |
| A.004 | $CH_3$ | C≡CH | 5–$COOCH_3$ |
| A.005 | $CH_3$ | C≡CH | 5–$COOCH_3$ |
| B.001 | $CH_3$ | C≡CH | 5–$COOCH_3$ |
| B.005 | $CH_3$ | C≡CH | 5–$COOCH_3$ |

Tabelle B

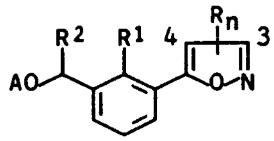

Ib

| A | $R^1$ | $R^2$ | $R_n$ |
|---|---|---|---|
| A.001 | $CH_3$ | H | $3-CH_2CH_3$ |
| A.002 | $CH_3$ | H | $3-CH_2CH_3$ |
| A.003 | $CH_3$ | H | $3-CH_2CH_3$ |
| A.004 | $CH_3$ | H | $3-CH_2CH_3$ |
| A.005 | $CH_3$ | H | $3-CH_2CH_3$ |
| B.001 | $CH_3$ | H | $3-CH_2CH_3$ |
| A.005 | $CH_3$ | H | $3-CH_2CH_3$ |
| A.001 | $CH_3$ | H | $3-CH_3$ |
| A.002 | $CH_3$ | H | $3-CH_3$ |
| A.003 | $CH_3$ | H | $3-CH_3$ |
| A.004 | $CH_3$ | H | $3-CH_3$ |
| A.005 | $CH_3$ | H | $3-CH_3$ |
| B.001 | $CH_3$ | H | $3-CH_3$ |
| A.005 | $CH_3$ | H | $3-CH_3$ |
| A.001 | $CH_3$ | H | - |
| A.002 | $CH_3$ | H | - |
| A.003 | $CH_3$ | H | - |
| A.004 | $CH_3$ | H | - |
| A.005 | $CH_3$ | H | - |
| B.001 | $CH_3$ | H | - |
| A.005 | $CH_3$ | H | - |

Die 3-Isoxazolyl-benzylester der Formel Ia und Ib sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana,

Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus

claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 2.001 werden mit 95 Gew.-Teilen feinteiligem Kaolin innig vermischt. Man erhält auf diese Weise ein Stäubemittel, das 5 Gew.% des Wirkstoffs enthält.

II. 30 Gew.-Teile der Verbindung Nr. 1.003 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).

III. 10 Gew.-Teile der Verbindung Nr. 1.002 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).

IV. 20 Gew.-Teile der Verbindung Nr. 1.005 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).

V. 80 Gew.-Teile der Verbindung Nr. 1.001 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).

VI. Man vermischt 90 Gew.-Teile der Verbindung Nr. 1.004 mit 10 Gew.-Teilen N-Methyl-$\alpha$-pyrrolidon und erhält eine Lösung, die zur Anwendung in Form Kleinster Tropfen geeignet ist (Wirkstoffgehalt 90 Gew.%).

VII. 20 Gew.-Teile der Verbindung Nr. 1.001 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

VIII. 20 Gew.-Teile des Wirkstoffs Nr. 2.001 werden mit 3 Gew.-Teilen des Natriumsalzes der Diisobutylnaphthalin-$\alpha$-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe Können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 10, vorzugsweise 0,1 bis 1 kg/ha.

Zu den Wirkstoffen Können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen Ia und Ib benutzt. Die so erhaltenen Verbindungen sind in den nachstehenden Tabellen mit physikalischen Angaben aufgeführt.

Herstellung von Ausgangsstoffen

1. 3-Amino-2-methylbenzylalkohol

Eine Lösung von 203 g 2-Methyl-3-nitrobenzylalkohol in 1 000 ml Ethanol wird in Gegenwart von 5 g Pd/Kohle (10%ig) bei 30 bis 35 °C hydriert. Der Katalysator wird abfiltriert, die Lösung eingeengt und der erhaltene Feststoff getrocknet. Man erhält 145 g 3-Amino-2-methylbenzylalkohol (Smp. 104 - 108 °C).

2. 3-Brom-2-methylbenzylalkohol

Zu einem auf 0 °C abgekühlten Gemisch von 888 ml Wasser, 162 ml Bromwasserstoffsäure (47%ig) und 81,2 g 3-Amino-2-methylbenzylalkohol wird eine Lösung von 40,7 g Natriumnitrat in 300 ml Wasser getropft. Man rührt 30 Minuten bei 0 °C und gibt dann bei dieser Temperatur portionsweise eine Suspension von 168,7 g Kupfer(I)-bromid in 750 ml Wasser hinzu. Das Reaktionsgemisch wird nacheinander 1 Stunde bei 10 °C, 1 Stunde bei Raumtemperatur und 2 Stunden bei 100 °C gerührt. Nach dem Abkühlen wird das Reaktionsgemisch mehrmals mit Ether extrahiert. Die vereinigten organischen Phasen werden mit Wasser gewaschen, getrocknet und eingeengt. Nach säulenchromatographischer Reinigung über Kieselgel mit Toluol als Fließmittel erhält man 64,2 g 3-Brom-2-methylbenzylalkohol (Smp. 97 - 100 °C).

3. (3-Brom-2-methylbenzyl)-(tetrahydro-2-pyranyl)ether

Zu einer Lösung von 208,9 g 3-Brom-2-methylbenzylalkohol und 87,4 g 3,4-Dihydro-2H-pyran in 1600 ml Ether werden bei 0 °C 2,1 ml konzentrierte Salzsäure gegeben. Man rührt 4 Tage lang bei Raumtempratur und tropft dann 50 ml 10%ige Kalilauge und 500 ml Wasser hinzu. Die organische Phase wird abgetrennt, die wäßrige Phase mit Ether extrahiert und die vereinigten organischen Phasen werden mit Wasser gewaschen, getrocknet und eingeengt. Nach säulenchromatographischer Reinigung über Kieselgel mit Toluol als Fließmittel erhält man 224,6 g der gewünschten Verbindung.

NMR-Spektrum [300-MHz; $CDCl_3$; δ (ppm)]:

1,4-1,91 (6H); 2,42 (3H); 3,55 (1H); 3,89 (1H); 4,47 (1H); 4,68 (1H); 4,8 (1H); 7,02 (1H); 7,32 (1H); 7,47 (1H).

4. (3-Formyl-2-methylbenzyl)-(tetrahydro-2-pyranyl)ether

Unter Stickstoffatmosphäre werden 1,2 g Mg (Magnesiumspäne) in 15 ml absolutem Tetrahydrofuran vorgelegt. Bei 65 °C fügt man einige Tropfen Dibromethan hinzu. Während man die Temperatur weiterhin bei 65 °C hält wird eine Lösung von 14,25 g (3-Brom-2-methylbenzyl)-(tetrahydro-2-pyranyl)ether in 50 ml Tetrahydrofuran zugetropft. Anschließend rührt man 2 Stunden unter Rückfluß. Zu der auf 0 °C abgekühlten Reaktionsmischung wird eine Lösung von 5,65 g N-Formylpiperidin in 10 ml absolutem Tetrahydrofuran getropft. Man rührt 20 Stunden bei Raumtemperatur, stellt mit ca. 50 ml 5%iger Salzsäure schwach sauer und extrahiert mehrmals mit Ether. Die vereinigten Etherextrakte werden mit Wasser gewaschen, getrocknet und eingeengt. Das Rohprodukt (11 g) kann säulenchromatographisch über Kieselgel mit Toluol/Aceton (97,5:2,5) gereinigt werden.

NMR-Spektrum [250-MHz; $CDCl_3$; δ (ppm)]:

1,48-1,93 (6H); 2,64 (3H); 3,57 (1H); 3,9 (1H); 4,54 (1H); 4,72 (1H); 4,86 (1H); 7,36 (1H); 7,65 (1H); 7,76 (1H); 10,33 (1H).

5. [3-(2',2'-Dibromvinyl)-2-methylbenzyl]-(tetrahydro-2-pyranyl)ether

Zu einer Lösung von 64,85 g Triphenylphosphin in 60 ml Methylenchlorid wird bei 0 °C eine Lösung von 41,44 g Tetrabrommethan in 40 ml Methylenchlorid getropft. Man rührt 30 Minuten bei 0 °C und tropft dann 23,1 g (3-Formyl-2-methylbenzyl)-(tetrahydro-2-pyranyl)ether in 25 ml Methylenchlorid hinzu. Nach 2stündigem Rühren bei Raumtemperatur wird der Feststoff abfiltriert und das Filtrat eingeengt. Zum Filtrat werden 200 ml Cyclohexan und 200 ml Wasser hinzugegeben. Man rührt 1 Stunde unter Rückfluß, trennt die organische Phase ab, trocknet und engt ein. Nach säulenchromatographischer Reinigung über Kieselgel mit Cyclohexan und Toluol als Fließmittel erhält man neben 4 g 3-(2',2'-Dibromvinyl)-2-methylbenzylbromid 18,4 g der gewünschten Verbindung.

NMR-Spektrum [300-MHz; $CDCl_3$; δ (ppm)]:

1,48 - 1,92 (6H); 2,23 (3H); 3,55 (1H); 3,9 (1H); 4,48 (1H); 4,72 (1H); 4,81 (1H); 7,19 (1H); 7,28 (1H); 7,38 (1H); 7,5 (1H).

6. [3-(2',2'-Dichlorvinyl)-2-methylbenzyl]-(tetrahydro-2-pyranyl)ether

Methode A:

Zu einer Suspension von 3,37 g Kalium-tert.-butylat in 50 ml Heptan werden unter Stickstoffatmosphäre bei 0-5 °C zügig 7,86 g Triphenylphosphin gegeben. Anschließend werden ebenfalls bei 0-5 °C 3,59 g Chloroform in 30 ml Heptan innerhalb von 1 Stunde zugetropft. Das entstandene tert.-Butanol wird bei 0 °C unter vermindertem Druck abdestilliert. Bei 5-10 °C tropft man innerhalb von 30 Minuten eine Lösung von 7,02 g (3-Formyl-2-methylbenzyl)-(tetrahydro-2-pyranyl)ether in 10 ml Heptan ein, rührt 2 Stunden bei 5 °C und 15 Stunden bei Raumtemperatur. Der ausgefallene Feststoff wird abfiltriert und die Lösung eingeengt. Nach säulenchromatographischer Reinigung über Kieselgel mit Toluol als Fließmittel erhält man 2,3 g des gewünschten Ethers.

Methode B:

Zu einer Lösung von 12,8 g Trichlormethyl-phosphonsäure-diethylester in 45 ml Ether und 35 ml Tetrahydrofuran werden unter Stickstoffatmosphäre bei -100 °C 33,1 ml (0,053 mol) n-Butyllithium (15 %ige Lösung in Hexan) und anschließend 11,7 g (3-Formyl-2-methylbenzyl)-(tetrahydro-2-pyranyl)ether in 10 ml Ether/Tetrahydrofuran (1:1) getropft. Unter Rühren läßt man auf Raumtemperatur aufwärmen und erhitzt 1 Stunde unter Rückfluß. Die Reaktionsmischung wird auf -50 °C abgekühlt, mit 50 ml 2N Schwefelsäure versetzt, in 300 ml Wasser gegossen und mehrmals mit Ether extrahiert. Die vereinigten Etherphasen werden mit Wasser gewaschen, getrocknet und eingeengt. Nach säulenchromatographischer Reinigung über Kieselgel mit Toluol als Fließmittel erhält man 4,7 g der gewünschten Verbindung.

NMR-Spektrum [300-MHz; $CDCl_3$; δ (ppm)]:

1,47-1,93 (6H); 2,25 (3H); 3,57 (1H); 3,92 (1H); 4,48 (1H); 4,72 (1H); 4,81 (1H); 6,95 (1H); 7,2 (1H); 7,35 (2H).

1. Synthese der 3-Isoxazolyl-benzylderivate IIIa

1.1 3-(3'-Isoxazolyl)-2-methylbenzylalkohol

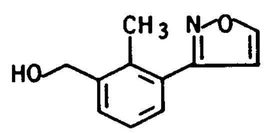

A (3-Hydroxyiminomethyl-2-methylbenzyl)-(tetrahydro-2-pyranyl)-ether

Eine Lösung aus 6,0 g (3-Formyl-2-methylbenzyl)-(tetrahydro-2-pyranyl)ether und 50 ml Toluol wurde bei 25 °C mit einer Lösung aus 2,67 g Hydroxylaminhydrochlorid und 10 ml Wasser versetzt. Nach der Zugabe von 2,01 g Natriumcarbonat in 10 ml $H_2O$ wurde über Nacht bei 25 °C gerührt. Im Verlauf der Reaktion auskristallisiertes Produkt wurde abfiltriert und in Ether gelöst. Aus den vereinigten organischen Phasen erhielt man nach Waschen und Trocknen 6,4 g Produkt.

NMR-Spektrum [300 MHz; $CDCl_3$; δ (ppm)]: 1,2-1,85 (6H); 2,32 (3H); 3,50 (1H); 3,78 (1H); 4,40-4,85 (3H); 7,05-7,7 (3H); 8,42 (1H); 11,27 (1H).

B (3-(3'-Isoxazolyl)-2-methylbenzyl)-(tetrahydro-2-pyranyl)-ether

In eine Lösung aus 6,23 g (3-Hydroxyiminomethyl-2-methylbenzyl)-(tetrahydro-2-pyranyl)ether und 50 ml $CH_2Cl_2$ wurde bei 0 bis 5 °C innerhalb 30 Minuten Acetylen eingeleitet. Anschließend wurden 20,6 ml einer 10%igen Natriumhypochlorit-Lösung versetzt mit einer Spatelspitze Natriumacetat bei 10 °C unter weiterer Acetylen-Einleitung zugetropft. Nach beendeter Zugabe wurde für weitere 15 Minuten Acetylen bei 10 °C eingeleitet. Danach wurde 1 Stunde bei 10 °C gerührt. Nach 14 Stunden bei 25 °C wurden die beiden Phasen getrennt. Aus der organischen Phase erhielt man nach Waschen, Trocknen und säulenchromatographischer Reinigung (Kieselgel; Toluol/Aceton 97,5:2,5) 4,8 g an Produkt.

NMR-Spektrum [250 MHz; $CDCl_3$; δ (ppm)]: 1,40-2,0 (6H); 2,38 (3H); 3,55 (1H); 3,92 (1H); 4,45-4,90 (3H); 6,48 (1H); 7,20-7,50 (3H); 8,43 (1H).

C 4,7 g (3-(3'-Isoxazolyl)-2-methylbenzyl)-(tetrahydro-2-pyranyl)ether gelöst in 40 ml Methanol wurden mit 2,72 ml konzentrierter Salzsäure für 14 Stunden bei 25 °C gerührt. Anschließend wurde unter Eiskühlung mit Natriummethanolat-Lösung neutralisiert und die neutrale Lösung bei vermindertem Druck eingeengt. Der Rückstand wurde mit Wasser versetzt und die Lösung wurde mehrmals mit Diethylether extrahiert. Aus den vereinigten Etherextrakten erhielt man 3,0 g 3-(3'-Isoxazolyl)-2-methylbenzylalkohol.

NMR-Spektrum [300 MHz; $CDCl_3$; δ (ppm)]: 2,25 (3H); 2,70 (1H); 4,65 (2H); 6,43 (1H); 7,15-7,50 (3H); 8,27 (1H).

2. Synthese der 3-Isoxazolyl-benzylderivate IIIb

2.1. 3-[5'-(3'-Ethylisoxazolyl)]-2-methylbenzylalkohol

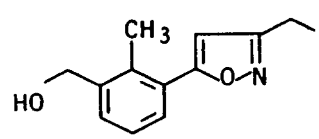

A (3-Ethinyl-2-methylbenzyl)-(tetrahydro-2-pyranyl)ether

Eine Lösung von 5,85 g (3-(2',2'-Dibromvinyl)-2-methylbenzyl)-(tetrahydro-2-pyranyl)ether und 50 ml Tetrahydrofuran wurde bei -78 °C mit 19,7 ml einer 1,6 molaren Lösung von n-Butyllithium in n-Hexan versetzt. Nach 1 h bei -78 °C wurde 1 h bei 25 °C belassen und anschließend wurde die Reaktionslösung zu 300 ml Eiswasser gegegeben. Die so erhaltene Mischung wurde mit Diethylether extrahiert. Aus den vereinigten organischen Phasen erhielt man nach Waschen, Trocknen und chromatographischer Reinigung (Kieselgel, Toluol) 3,1 g des Produkts (Reinheitsgrad von 80 % NMR).

NMR-Spektrum [200 MHz; $CDCl_3$; δ (ppm)]: 1,40-2,00 (6H); 2,48 (3H); 3,30 (1H); 3,60 (1H); 3,95 (1H); 4,40-4,95 (3H); 7,1-7,55 (3H).

B (3-[5'-(3'-Ethylisoxazolyl)]-2-methylbenzyl)-(tetrahydro-2 -pyranyl)ether

Ein Gemisch aus 9,2 g Phenylisocyanat, 10,6 g (3-Ethinyl-2-methylbenzyl)-(tetrahydro-2-pyranyl)-ether und 20 ml Toluol wurde bei 25 °C mit einer Lösung aus 3,43 g Nitropropan und 10 ml Toluol und mit 3 Tropfen Triethylamin versetzt. Nach 14 h bei 25 °C wurde für 2 h auf 100 °C erhitzt. Nach Abkühlung auf 25 °C wurde die Reaktionsmischung von festen Bestandteilen befreit. Aus der so erhaltenen Lösung erhielt man nach Chromatographie [Kieselgel ; Toluol/Aceton 98:2] 2,3 g des Produktes.

NMR-Spektrum [300 MHz; $CDCl_3$; δ (ppm)]: 1,36 (3H); 1,42-1,95 (6H); 2,21 (3H); 2,75 (2H); 3,55 (1H); 3,92 (1H); 4,4-49 (3H); 6,23 (1H); 7,0-7,6 (3H).

C 2,3 g 3-[5'-(3'-Ethylisoxazolyl)]-2-methylbenzyl-(tetrahydro-2-pyranyl)ether gelöst in 40 ml Methanol wurden mit 1,22 ml konzentrierter Salzsäure für 14 Stunden bei 25 °C gerührt. Anschließend wurde unter Eiskühlung mit Natriummethanolat-Lösung neutralisiert und die neutrale Lösung bei vermindertem Druck eingeengt. Der Rückstand wurde mit Wasser versetzt und die Lösung wurde mehrmals mit Diethylether extrahiert. Aus den vereinigten Etherextrakten erhielt man 1,4 g 3-[5'-(3'-Ethylisoxazolyl)]-2-methylbenzylalkohol.

NMR-Spektrum [300 MHz; $CDCl_3$, δ (ppm)]: 1,33 (2H); 2,37 (3H); 3,75 (2H); 4,72 (2H); 6,21 (1H); 7,2-7,6 (3H)

3. Synthese der 3-Isoxazolyl-benzylester Ia und Ib

3.1 cis,trans-3-(2'-Chlor-3',3',3'-trifluorprop-1'-enyl)-2,2-dimethylcyclopropan-1-carbonsäure-(3-(3'-isoxazolyl)-2-methyl benzylester

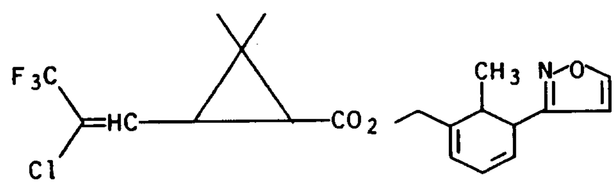

Eine Mischung aus 3,0 g 3-(3'-Isoxazolyl)-2-methylbenzylalkohol, 1,67 g Picolin und 30 ml Tetrahydrofuran wurde bei 20 °C tropfenweise mit 4,4 g 3-(2'-Chlor-3',3',3'-trifluorprop-1'-enyl)-2,2-dimethylcyclopropa n-1-carbonsäurechlorid (cis/trans = 1:1) versetzt. Nach dem Abklingen der exothermen Reaktion und weiteren 5 h Rühren wurde die Reaktionsmischung von festen Rückständen befreit. Aus der Lösung erhielt man 5,1 g Produkt.

NMR-Spektrum [200 MHz; $CDCl_3$; δ (ppm)]: 1,20-1,40 (6H); 1,83; 2,02; 2,18; 2,37 (Σ 2H); 2,40 (3H); 5,10 (2H); 6,08; 6,95 (Σ 1H); 6,60 (1H); 7,20-7,5 (3H); 8,50 (1H). Wirkstoffbeispiel 1.005

Tabelle 1

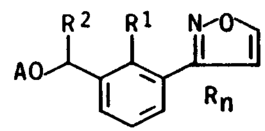

Ia

| Beispiel Nr. | A | $R^1$ | $R^2$ | $R_n$ | physik. Daten [Fp. (°C); NMR (δ in ppm)] | Isomer (A-OH) |
|---|---|---|---|---|---|---|
| 1.001 | A.002 | $CH_3$ | H | - | (300 MHz; $CDCl_3$) 1,08-1,40 (6H); 1,67-2,33 (2H); 2,38 (3H); 5,20 (2H); 5,63 (0,5H); 6,28 (0,5H); 6,49 (1H); 7,25-7,58 (3H); 8,47 (1H) | Gemisch |
| 1.002 | A.004 | $CH_3$ | H | - | (300 MHz; $CDCl_3$) 1,07-1,42 (6H); 1,65-2,37 (2H); 2,40 (3H); 5,20 (2H); 6,17 (0,9H); 6,48 (1H); 6,80 (0,1H); 7,1-7,6 (3H); 8,45 (1H) | Gemisch |
| 1.003 | A.005 | $CH_3$ | H | - | (200 MHz; $CDCl_3$) 1,1-1,3 (6H); 1,42-2,15 (2H); 2,38 (3H); 3,95-4,80 (1H); 5,18 (2H); 6,50 (1H); 7,20-7,57 (3H); 8,50 (1H) | Gemisch |
| 1.004 | B.001 | $CH_3$ | H | - | (300 MHz; $CDCl_3$) 0,70 (3H); 1,02 (3H); 2,10-2,50 (1H); 2,25 (3H); 3,18 (1H); 5,17 (2H); 6,43 (1H); 7,10-7,55 (7H); 8,47 (1H) | Gemisch |

Tabelle 1 - Fortsetzung

| Beispiel Nr. | A | $R^1$ | $R^2$ | $R_n$ | physik. Daten [Fp. (°C); NMR (δ in ppm)] | Isomer (A-OH) |
|---|---|---|---|---|---|---|
| 1.005 | A.003 | $CH_3$ | H | - | (200 MHz; $CDCl_3$) 1,20-1,40 (6H); 1,83; 2,02; 2,18; 2,37 (2H); 2,40 (3H); 5,10 (2H); 6,08; 6,95 (1H); 6,60 (1H); 7,20-7,5 (3H); 8,50 (1H) | Gemisch |
| 1.006 | A.003 | $CH_3$ | H | - | (200 MHz; $CDCl_3$) 1,30 (6H); 1,95-2,30 (2H); 2,40 (3H); 5,17 (2H); 6,5 (1H); 6,93 (1H); 7,20-7,50 (3H); 8,50 (1H); | cis* |
| 1.007 | A.003 | $CH_3$ | H | - | (300 MHz; $CDCl_3$) 1,15-1,40 (6H); 1,75-1,85 (2H); 2,40 (3H); 5,22 (2H); 5,90-6,15 (1H); 6,50 (1H); 7,20-7,60 (3H); 8,50 (1H) | trans* |

*bezügl. Stereochemie am Cyclopropanring

Tabelle 2

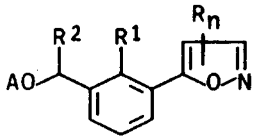

Ib

| Beispiel Nr. | A | $R^1$ | $R^2$ | $R_n$ | physik. Daten [Fp. (°C); NMR (δ in ppm)] | Isomer (A-OH) |
|---|---|---|---|---|---|---|
| 2.001 | A.002 | $CH_3$ | H | $3-CH_2CH_3$ | (300 MHz; $CDCl_3$) 1,10-1,50 (9H); 1,80-2,40 (2H); 2,38 (3H); 3,78 (2H); 5,21 (2H); 6,18 (0,5H); 6,24 (1H); 6,90 (0,5H); 7,20-7,60 (3H); | Gemisch |

Anwendungsbeispiele

Die insektizide Wirkung der 3-Isoxazolylbenzylester der allgemeinen Formeln Ia und Ib ließ sich durch folgende Versuche zeigen:

Die Wirkstoffe wurden

a) als 0,1 %ige Lösung in Aceton oder

b) als 10 %ige Emulsion in einem Gemisch aus 70 Gew.-% Cyclohexanol, 20 Gew.-% Nekanil® LN (Lutensol® AP6, Netzmittel mit Emulgier- und Dispergierwirkung auf Basis ethoxylierter Alkylphenole) und 10 Gew.-% Emulphor® EL (Emulan® EL, Emulgator auf Basis ethoxylierter Fettalkohole)

aufbereitet und entsprechend der gewünschten Konzentration mit Aceton im Falle a) bzw. mit Wasser im Falle von b) verdünnt.

Beispiel A

Blatta orientalis (orientalische Schabe)

Kontaktwirkung

Der Boden eines 1 l-Glases (Φ ca. 10 cm) wurde mit einer acetonischen Lösung des Wirkstoffes bedeckt. Nach dem Abdampfen des Lösungsmittels wurden 5 adulte Schaben in das Glas gesetzt. Nach 48 Stunden wurde die Mortalitätsrate in % bestimmt.

In diesem Versuch zeigten die Verbindungen Nr. 1.001, Nr. 1.002 Nr. 1.003, Nr. 1.005 und Nr. 2.001 bei einer Wirkstoffkonzentration von 0,04 bis 1 mg Mortalitätsraten von 80 bis 100 %.

Beispiel B

Plutella maculipennis (Kohlenschaben-Raupe)

Kontaktwirkung

Blätter junger Kohlpflanzen wurden kurzzeitig (3 Sek.) in die wäßrige Wirkstoffemulsion getaucht und nach dem Abtropfen auf einen angefeuchteten Filter in eine Petrischale gelegt. Die so vorbereiteten Blätter wurden pro Petrischale mit 10 Raupen des 4. Entwicklungsstadiums belegt. Nach 48 Stunden wurde die Mortalitätsrate in % bestimmt.

In diesem Versuch zeigten die Verbindungen Nr. 1.001, Nr. 1.002, Nr. 1.003, Nr. 1.004 und Nr. 2.001 bei einer Wirkstoffkonzentration von 20 bis 1000 ppm Mortalitätsraten von 60 bis 100 %.

Beispiel C

Tetranychus telarius (Rote Spinne)

Kontaktwirkung

Stark befallene getopfte Buschbohnen, die das zweite Folgeblattpaar zeigten, wurden mit der wäßrigen Wirkstoffaufbereitung tropfnaß gespritzt. Nach 5 Tagen im Gewächshaus wurde mittels Binokular der Bekämpfungserfolg in % festgestellt.

In diesem Versuch zeigten die Verbindungen Nr. 1.001, Nr. 1.002, Nr. 1.003, Nr. 1.004, Nr. 1.005 und Nr. 2.001 bei einer Wirkstoffkonzentration von 4 bis 1000 ppm einen Bekämpfungserfolg von 80 bis 100 %.

## Patentansprüche

1. 3-Isoxazolylbenzylester der allgemeinen Formeln Ia und Ib

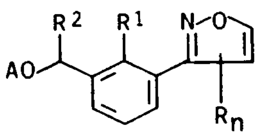

Ia

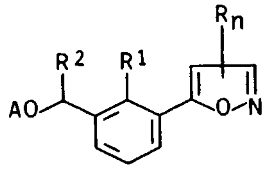

Ib

in denen die Substituenten und der Index folgende Bedeutung haben:

| | |
|---|---|
| R | Halogen; $C_1$-$C_4$-Alkyl; $C_1$-$C_4$-Halogenalkyl; $C_1$-$C_4$-Alkoxy; $C_1$-$C_4$-Halogenalkoxy; $C_2$-$C_4$-Alkenyl; $C_2$-$C_4$-Halogenalkenyl ; Phenylethenyl, welches ein bis fünf Halogenatome tragen kann; $C_2$-$C_4$-Alkinyl; $C_3$-$C_8$-Cycloalkyl; Phenyl, Naphthyl; 5- oder 6-gliedriges Hetaryl; $CO_2R^3$ oder $CONR^4R^5$, wobei |
| $R^3$, $R^4$ und $R^5$ | Wasserstoff oder $C_1$-$C_6$-Alkyl bedeuten; |
| n | 0, 1 oder 2, wobei die Reste R verschieden sein können, wenn n 2 bedeutet; |
| $R^1$ | Halogen oder $C_1$-$C_4$-Alkyl ; |
| $R^2$ | Wasserstoff; $C_1$-$C_4$-Alkyl ; $C_2$-$C_4$-Alkenyl; $C_2$-$C_4$-Alkinyl oder Cyano ; |
| A | der Carbonylrest einer Pyrethroidsäure der allgemeinen Formeln IIa oder IIb |

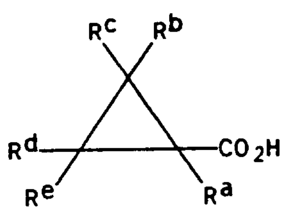

IIa

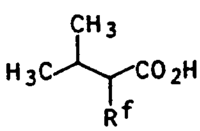

IIb

in denen die Substituenten folgende Bedeutung haben:

| | |
|---|---|
| $R^a$ | Wasserstoff, $C_1$-$C_4$-Alkyl oder Phenyl, wobei der aromatische Ring ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio; |
| $R^b$, $R^c$, $R^d$ | Wasserstoff; Halogen oder $C_1$-$C_4$-Alkyl ; |
| $R^e$ | Wasserstoff; Halogen; $C_1$-$C_4$-Alkyl; $C_1$-$C_4$-Halogenalkyl; $C_2$-$C_6$-Alkenyl oder $C_2$-$C_6$-Alkdienyl, welche ein bis acht Halogenatome und/oder einen der folgenden Reste tragen können: $C_1$-$C_4$-Alkoxycarbonyl oder Phenyl, wobei der Phenylrest seinerseits ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio; $C_3$-$C_7$-Cycloalkylidenmethyl oder |
| $R^d$ und $R^e$ | gemeinsam eine 2, 2H-Indenspirogruppe; |

$R^f$ ein ein- oder zweikerniges aromatisches oder heteroaromatisches Ringsystem, welches als Heteroatom ein Stickstoffatom enthalten kann, wobei dieses Ringsystem ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio oder
eine Phenylaminogruppe, wobei der Phenylring seinerseits ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio.

**2.** 3-Isoxazolyl-benzylester der allgemeinen Formel Ia und Ib gemäß Anspruch 1, dadurch gekennzeichnet, daß $R^1$ Methyl oder Ethyl und $R^2$ Wasserstoff bedeuten.

**3.** 3-Isoxazolyl-benzylester der allgemeinen Formel Ia und Ib gemäß Anspruch 1, dadurch gekennzeichnet, daß $R^1$ Methyl oder Ethyl, $R^2$ Wasserstoff und n 0 bedeuten.

**4.** 3-Isoxazolyl-benzylester der allgemeinen Formeln Ia und Ib gemäß Anspruch 1, dadurch gekennzeichnet, daß A für den Carbonylrest einer Pyrethroidsäure der allgemeinen Formel IIa steht, in der
$R^a$ und $R^d$ Wasserstoff
$R^b$ und $R^c$ Methyl und
$R^e$ $C_2$-$C_4$-Alkenyl, welches zwei bis sechs Halogenatome trägt
bedeutet.

**5.** 3-Isoxazolylbenzylderivate der allgemeinen Formeln IIIa und IIIb

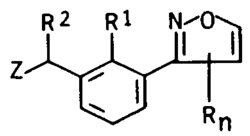

IIIa

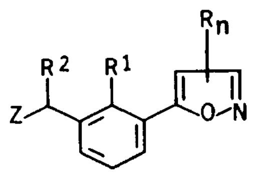

IIIb

in der die Substituenten R, $R^1$ und $R^2$ und der Index n die in Anspruch 1 angegebene Bedeutung haben und Z für Hydroxy Oder Halogen steht.

**6.** Verfahren zur Herstellung der Verbindungen Ia und Ib gemäß Anspruch 1, dadurch gekennzeichnet, daß man ein 3-Isoxazolylbenzylderivat der allgemeinen Formel IIIa oder IIIb gemäß Anspruch 5 in an sich bekannter Weise mit einer bei Pyrethroiden üblichen Carbonsäure A-OH oder deren Derivaten umsetzt.

**7.** Verfahren zur Herstellung der Verbindungen IIIa gemäß Anspruch 5, in denen $R^2$ Wasserstoff und Z Hydroxy bedeutet, dadurch gekennzeichnet, daß man einen geschützten 3-Formylbenzylalkohol der allgemeinen Formel IV

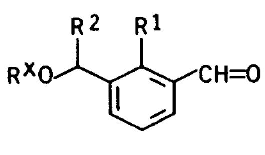

IV

in der $R^x$ für eine Schutzgruppe steht, in an sich bekannter Weise in das entsprechende Oxim Va

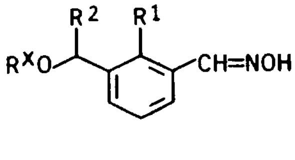

Va

überführt, Va anschließend in Gegenwart eines Oxidationsmittels an ein Alkin der Formel VIa

$R_m$ - ≡ - $R_p$     VIa

in dem die Reste R verschieden sein können und m und p 0 oder 1 bedeuten, addiert und aus dem so erhaltenen geschützten 3-Isoxazolylbenzylalkohol VIIa

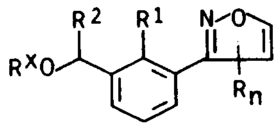

VIIa

in an sich bekannter Weise die Schutzgruppe abspaltet.

**8.** Verfahren zur Herstellung der Verbindungen IIIb gemäß Anspruch 5, in denen $R^2$ Wasserstoff und Z Hydroxy bedeutet, dadurch gekennzeichnet, daß man einen geschützten 3-Formylbenzylalkohol der allgemeinen Formel IV gemäß Anspruch 7 in an sich bekannter Weise mittels eines Phosphonium- oder Phosphonat-Reagens in einer Wittig- bzw. Horner-Wittig-Reaktion in das entsprechende 3-Bromvinylderivat VIII

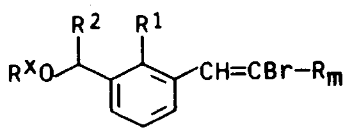

VIII

überführt, VIII anschließend in an sich bekannter Weise in Gegenwart einer Base in ein Alkin der Formel VIb

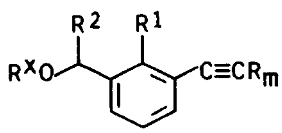

VIb

in der R und m die in Anspruch 7 gegebene Bedeutung haben, überführt, welches danach in an sich bekannter Weise in Gegenwart eines Oxidationsmittels mit einem Oxim der Formel Vb

$R_p$-C = NOH     Vb

zum 3-Isoxazolyl-benzylether VIIb

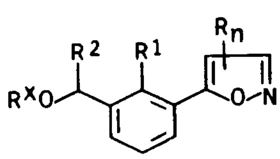

VIIb

umgesetzt wird, aus dem in an sich bekannter Weise gemäß Anspruch 7 die Schutzgruppe abgespalten wird.

**9.** Verfahren zur Herstellung der Verbindungen IIIa und IIIb gemäß Anspruch 5, in denen $R^2$ nicht Wasserstoff und Z Hydroxy bedeutet, dadurch gekennzeichnet, daß man einen 3-Isoxazolylbenzylalkohol der allgemeinen Formel IIIa oder IIIb (Z = OH) in dem $R^2$ Wasserstoff bedeutet in an sich bekannter Weise oxidiert und den so erhaltenen Benzaldehyd IXa bzw. IXb

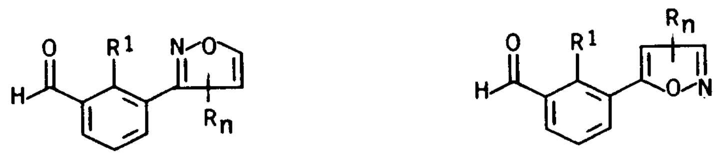

IXa IXb

mit einer metallorganischen Verbindung $MR^2$, worin M für ein Alkalimetall-, ein Erdalkalimetall- oder ein Übergangsmetall steht und $R^2$ nicht Wasserstoff bedeutet, in an sich bekannter Weise umsetzt.

**10.** Verfahren zur Herstellung der Verbindungen Ia und Ib gemäß Anspruch 1, in denen $R^2$ Cyano bedeutet, dadurch gekennzeichnet, daß man das Halogenid einer Pyrethroidsäure in an sich bekannter Weise in Gegenwart eines Alkalimetall- oder Erdalkalimetallcyanids mit einem 3-Isoxazolylbenzaldehyd der allgemeinen Formel IXa bzw. IXb gemäß Anspruch 9 umsetzt.

**11.** Mittel zur Bekämpfung von Schädlingen, enthaltend einen 3-Isoxazolylbenzylester der allgemeinen Formel Ia oder Ib gemäß Anspruch 1 und inerte Zusatzstoffe.

**12.** Verfahren zur Bekämpfung von Schädlingen, dadurch gekennzeichnet, daß man die Schädlinge und/oder deren Lebensraum mit einer wirksamen Menge eines 3-Isoxazolylbenzylesters der Formel Ia oder Ib gemäß Anspruch 1 behandelt.

## Claims

**1.** A 3-isoxazolylbenzyl ester of the formula Ia or Ib

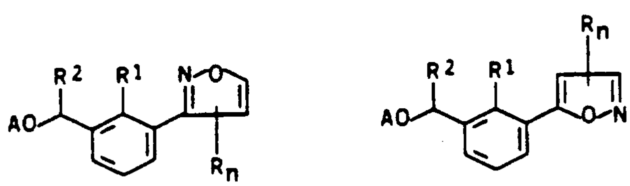

Ia Ib

where

R is halogen, $C_1$-$C_4$-alkyl, $C_1$-$C_4$-haloalkyl, $C_1$-$C_4$-alkoxy, $C_1$-$C_4$-haloalkoxy, $C_2$-$C_4$-alkenyl or $C_2$-$C_4$-haloalkenyl or is phenylethenyl which may carry from one to five halogen atoms, or is $C_2$-$C_4$-alkynyl, $C_3$-$C_8$-cycloalkyl, phenyl, naphthyl, 5-membered or 6-membered hetaryl, $CO_2R^3$ or $CONR^4R^5$,
$R^3$, $R^4$ and $R^5$ are each hydrogen or $C_1$-$C_6$-alkyl,
n is 0, 1 or 2, and the radicals R may be different when n is 2,
$R^1$ is halogen or $C_1$-$C_4$-alkyl,
$R^2$ is hydrogen, $C_1$-$C_4$-alkyl, $C_2$-$C_4$-alkenyl, $C_2$-$C_4$-alkynyl or cyano and
A is the carbonyl radical of a pyrethroid acid of the formula IIa or IIb

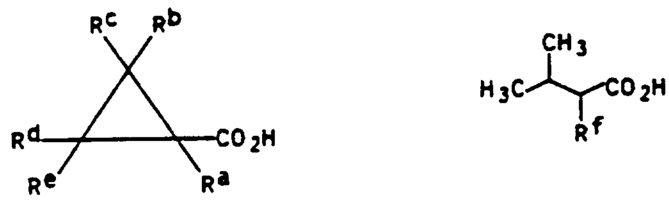

IIa IIb

where

$R^a$ is hydrogen, $C_1$-$C_4$-alkyl or phenyl, where the aromatic ring may carry from one to five halogen atoms and/or from one to three of the following radicals: $C_1$-$C_4$-alkyl, $C_1$-$C_4$-haloalkyl, $C_1$-$C_4$-alkoxy, $C_1$-$C_4$-haloalkoxy or $C_1$-$C_4$-alkylthio, $R^b$, $R^c$ and $R^d$ are each hydrogen, halogen or $C_1$-$C_4$-alkyl, $R^e$ is hydrogen, halogen, $C_1$-$C_4$-alkyl or $C_1$-$C_4$-haloalkyl or is $C_2$-$C_6$-alkenyl or $C_2$-$C_6$-alkadienyl which may carry from one to eight halogen atoms and/or one of the following radicals: $C_1$-$C_4$-alkoxycarbonyl or phenyl, where the phenyl radical in turn may carry from one to five halogen atoms and/or from one to three of the following groups: $C_1$-$C_4$-alkyl, $C_1$-$C_4$-haloalkyl, $C_1$-$C_4$-alkoxy, $C_1$-$C_4$-haloalkoxy or $C_1$-$C_4$-alkylthio, or $R^e$ is $C_3$-$C_7$-cycloalkylidenemethyl, or

$R^d$ and $R^e$ together form a 2,2H-indenespiro group and

$R^f$ is a mononuclear or dinuclear aromatic or heteroaromatic ring system which may contain a nitrogen atom as the hetero atom, where this ring system may carry from one to five halogen atoms and/or from one to three of the following radicals: $C_1$-$C_4$-alkyl, $C_1$-$C_4$-haloalkyl, $C_1$-$C_4$-alkoxy, $C_1$-$C_4$-haloalkoxy or $C_1$-$C_4$-alkylthio, or

a phenylamino group where the phenyl ring in turn may carry from one to five halogen atoms and/or from one to three of the following radicals: $C_1$-$C_4$-alkyl, $C_1$-$C_4$-haloalkyl, $C_1$-$C_4$-alkoxy, $C_1$-$C_4$-haloalkoxy or $C_1$-$C_4$-alkylthio.

2. A 3-isoxazolylbenzyl ester of the formula Ia or Ib as claimed in claim 1, wherein $R^1$ is methyl or ethyl and $R^2$ is hydrogen.

3. A 3-isoxazolylbenzyl ester of the formula Ia or Ib as claimed in claim 1, wherein $R^1$ is methyl or ethyl, $R^2$ is hydrogen and n is 0.

4. A 3-isoxazolylbenzyl ester of the formula Ia or Ib as claimed in claim 1, wherein A is the carbonyl radical of a pyrethroid acid of the formula IIa where $R^a$ and $R^d$ are each hydrogen, $R^b$ and $R^c$ are each methyl and $R^e$ is $C_2$-$C_4$-alkenyl which may carry from two to six halogen atoms.

5. A 3-isoxazolylbenzyl derivative of the formula IIIa or IIIb

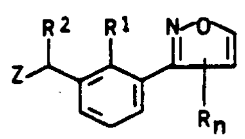

IIIa

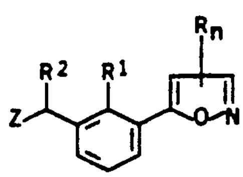

IIIb

where R, $R^1$ and $R^2$ and n have the meanings stated in claim 1 and Z is hydroxyl or halogen.

6. A process for the preparation of a compound Ia or Ib as claimed in claim 1, wherein a 3-isoxazolylbenzyl derivative of the formula IIIa or IIIb as claimed in claim 5 is reacted in a conventional manner with a carboxylic acid A-OH usually found in pyrethroids or with a derivative thereof.

7. A process for the preparation of a compound IIIa as claimed in claim 5, in which $R^2$ is hydrogen and Z is hydroxyl, wherein a protected 3-formylbenzyl alcohol of the formula IV

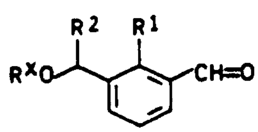

IV

where $R^x$ is a protective group, is converted in a conventional manner into the corresponding oxime Va

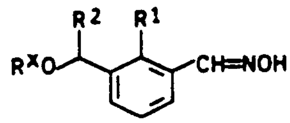

Va

Va is then subjected to an addition reaction with an alkyne of the formula VIa

$R_m - \equiv - R_p$    VIa

where the radicals R may be different and m and p are each 0 or 1, in the presence of an oxidizing agent, and the protective group is eliminated in a conventional manner from the resulting protected 3-isoxazolylbenzyl alcohol VIIa

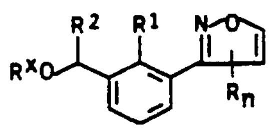

VIIa

**8.** A process for the preparation of the compound IIIb as claimed in claim 5, in which $R^2$ is hydrogen and Z is hydroxyl, wherein a protected 3-formylbenzyl alcohol of the formula IV as claimed in claim 7 is converted into the corresponding 3-bromovinyl derivative VIII

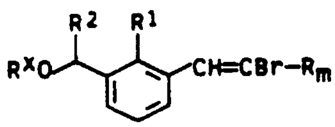

VIII

in a conventional manner by Wittig or Horner-Wittig reaction by means of a phosphonium or phosphonate reagent, VIII is then converted into an alkyne of the formula VIb

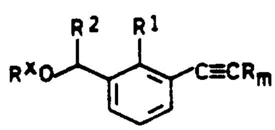

VIb

where R and m have the meanings stated in claim 7, in a conventional manner in the presence of a base, and said alkyne is then reacted in a conventional manner, in the presence of an oxidizing agent, with an oxime of the formula Vb

$R_p$-C = NOH    Vb

to give a 3-isoxazolylbenzyl ether VIIb

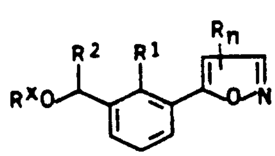

VIIb

from which the protective group is eliminated in a conventional manner as claimed in claim 7.

**9.** A process for the preparation of a compound IIIa or IIIb as claimed in claim 5, in which $R^2$ is not hydrogen and Z is hydroxyl, wherein a 3-isoxazolylbenzyl alcohol of the formula IIIa or IIIb (Z = OH), where $R^2$ is hydrogen, is oxidized in a conventional manner and the resulting benzaldehyde IXa or IXb

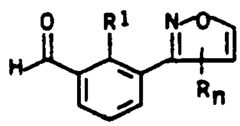

IXa

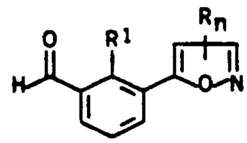

IXb

is reacted in a conventional manner with an organometallic compound $MR^2$, where M is an alkali metal, an alkaline earth metal or a transition metal and $R^2$ is not hydrogen.

**10.** A process for the preparation of a compound Ia or Ib as claimed in claim 1, in which $R^2$ is cyano, wherein the halide of a pyrethroid acid is reacted with a 3-isoxazolylbenzaldehyde of the formula IXa or IXb as claimed in claim 9 in a conventional manner in the presence of an alkali metal cyanide or alkaline earth metal cyanide.

**11.** A pesticide containing a 3-isoxazolylbenzyl ester of the formula Ia or Ib as claimed in claim 1 and inert additives.

**12.** A method for controlling pests, wherein the pests and/or their habitat are or is treated with an effective amount of a 3-isoxazolylbenzyl ester of the formula Ia or Ib as claimed in claim 1.

**Revendications**

**1.** 3-isoxazolylbenzylester des formules générales I a et Ib

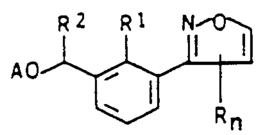

I a

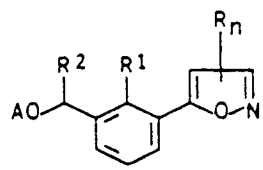

Ib

dans lesquelles les substituants et les indices ont les significations suivantes :
R, halogène, alkyle en C1-C4, halogénalkyle en C1-C4, alcoxy en C1-C4, halogénalcoxy en C1-C4, alcényle en C1-C4, halogénalcényle en C1-C4, phényléthényle qui peut porter un à cinq atomes d'halogène, alcynyle en C2-C4, cycloalkyle en C3-C8, phényle, naphtyle, hétaryle à 5 ou 6 chaînons, $CO_2R^3$ ou $CONR^4R^5$,
$R^3$, $R^4$ et $R^5$ représentant hydrogène ou alkyle en C1-C6,
n représentant 0, 1 ou 2, les restes R pouvant être différents quand n est mis pour 2,
$R^1$ halogène ou alkyle en C1-C4,
$R^2$ hydrogène, alkyle en C1-C4, alcényle en C2-C4, alcynyle en C2-C4 ou cyano,
A le reste carbonyle d'un acide pyrethroide des formules générales IIa ou IIb

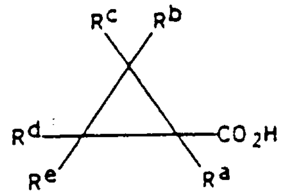

IIa

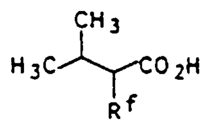

IIb

dans lesquelles les substituants ont la signification suivante :

$R^a$, hydrogène, alkyle en C1-C4 ou phényle, le noyau aromatique pouvant porter un à cinq atomes d'halogène et/ou une à trois des restes suivants : alkyle en C1-C4, halogénalkyle en C1-C4, alcoxy en C1-C4, halogenalcoxy en C1-C4 ou alkylthio en C1-C4,

$R^b$, $R^c$, $R^d$ hydrogène, halogène ou alkyle en C1-C4,

$R^e$ hydrogène, halogène, alkyle en C1-C4, halogénalkyle en C1-C4, alcényle en C2-C6 ou alkyldiényle en C2-C6, qui peuvent porter un à huit atomes d'halogène et/ou un des restes suivants : alcoxy en C1-C4-carbonyle ou phényle, le reste phényle pouvant porter, à son tour, un à cinq atomes d'halogène et/ou trois des groupes suivants : alkyle en C1-C4, halogénalkyle en C1-C4, alcoxy en C1-C4, halogénalcoxy en C1-C4 ou alkylthio en C1-C4, cycloalkyle en C3-C7-idènméthyle ou

$R^d$ et $R^e$, ensemble un groupe 2,2H-indenespiro,

$R^f$, un système cyclique aromatique ou hétéroaromatique à un ou deux noyaux, qui peut contenir comme hétéroatome un atome d'azote, ce système cyclique pouvant porter un à cinq atomes d'halogène et/ou un à trois des restes suivants : alkyle en C1-C4, halogénalkyle en C1-C4, alcoxy en C1-C4, halogénalcoxy en C1-C4 ou alkylthio en C1-C4 ou

un groupe phénylamino, le noyau phényle pouvant porter, à son tour, un à cinq atomes d'halogène et/ou un à trois des restes suivants :alkyle en C1-C4, halogénalkyle en C1-C4, alcoxy en C1-C4, halogénalcoxy en C1-C4 ou alkylthio en C1-C4.

**2.** 3-isoxazolylbenzylester des formules générales I a et Ib selon la revendication 1, caractérisé par le fait que $R^1$ représente méthyle ou éthyle et $R^2$, hydrogène.

**3.** 3-isoxazolylbenzylester des formules générales I a et Ib selon la revendication 1, caractérisé par le fait que $R^1$ représente méthyle ou éthyle, $R^2$, hydrogène et n représente 0.

**4.** 3-isoxazolylbenzylester des formules générales I a et Ib selon la revendication 1, caractérisé par le fait que A est mis pour le reste carbonyle d'un acide pyrethroide de la formule générale IIa, dans laquelle
$R^a$ et $R^d$ représentent hydrogène
$R^b$ et $R^c$ méthyle et
$R^e$, représente alcényle en C2-C4 qui porte deux à six atomes d'halogène.

**5.** Dérivés de 3-isoxazolylbenzyle des formules générales III a et IIIb

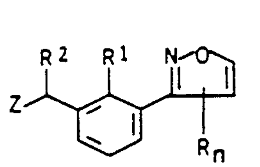

IIIa

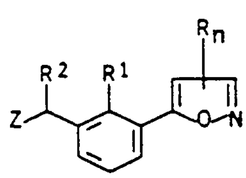

IIIb

dans lesquelles les substituants R, $R^1$ et $R^2$ et l'indice n ont la signification donnée dans la revendication 1 et Z est mis pour hydroxy ou halogène.

**6.** Procédé de préparation des composés Ia et Ib selon la revendication 1, caractérisé par le fait que l'on fait réagir de manière connue en soi un dérivé de 3-isoxazolylbenzyle des formules générales III a et IIIb selon la revendication 5, avec des acides carboxylique A-OH ou leurs dérivés, usuels chez les

pyrethroides.

**7.** Procédé de préparation de composés IIIa et IIIb selon la revendication 5 dans lesquels $R^2$ est mis pour hydrogène et Z pour hydroxy, caractérisé par le fait que l'on transforme, de manière connue en soi, un 3-formylbenzylalcool protégé, de la formule générale IV

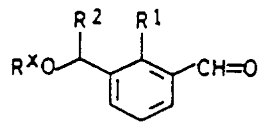

IV

dans laquelle $R^x$ est mis pour un groupe protecteur, en l'oxime Va correspondant

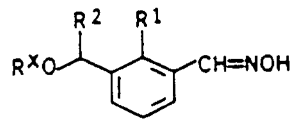

Va

on ajoute ensuite Va, en présence d'un agent d'oxydation, à une alkine de la formule VIa

$R_m$ - ≡ - $R_p$ $VI_a$

dans laquelle les restes R peuvent être différents et m et p représentent 0 ou 1, et on sépare, de manière connue en soi, le groupe protecteur, du 3-isoxazolylbenzylalcool VIIa protégé ainsi obtenu

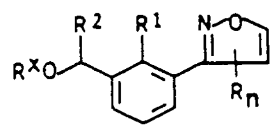

VIIa

**8.** Procédé de préparation des composés IIIa selon la revendication 5, dans lesquels $R^2$ représente hydrogène et Z hydroxy, caractérisé par le fait que l'on transforme, de manière connue en soi, un 3-formylbenzylalcool protégé de la formule générale IV selon la revendication 7, au moyen d'un réactif phosphonium ou phosphonate, dans une réaction de Wittig ou Horner-Wittig, en le dérivé de 3-bromovinyle VIII

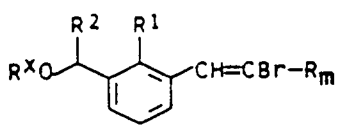

VIII

on transforme ensuite VIII, de manière connue en soi, en présence d'une base, en un alkine de la formule VIb

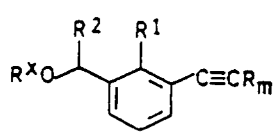

VIb

dans laquelle R et m ont la signification donnée dans la revendication 7, que l'on transforme ensuite, de manière connue en soi, avec un oxime de la formule Vb

$R_p$ - C = NOH Vb

en 3-isoxazolylbenzyléther VIIb

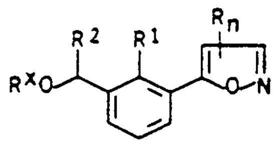

VIIb

duquel on sépare le groupe protecteur, de manière connue en soi, selon la revendication 7.

**9.** Procédé de préparation des composés IIIa et IIIb selon la revendication 5, dans lesquelles $R^2$ ne représente pas hydrogène et Z représente hydroxy, caractérisé par le fait que l'on oxyde de manière connue en soi, un 3-isoxazolylbenzylalcool des formules générales IIIa ou IIIb (Z = OH) dans lesquelles $R^2$ représente hydrogène et on fait réagir de manière connue en soi le benzaldéhyde IXa ou IXb ainsi obtenu

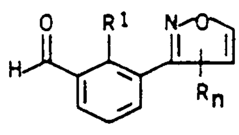

IXa

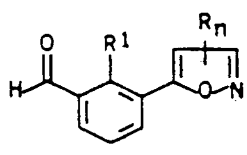

IXb

avec un composé organométallique $MR^2$ où M est mis pour un métal alcalin, alcalinoterreux ou de transition et $R^2$ ne représente pas hydrogène.

**10.** Procédé de préparation des composés Ia et Ib selon la revendication 1 dans lesquels R représente cyano, caractérisé par le fait que l'on fait réagir l'halogénure d'un acide pyrethroide, de manière connue en soi, en présence d'un cyanure de métal alcalin ou alcalinoterreux, avec un 3-isoxazylbenzaldéhyde des formules générales IXa ou IXb selon la revendication 9.

**11.** Moyen de lutte contre les parasites, contenant un 3-isoxazolylbenzyléther des formules générales Ia ou Ib selon la revendication 1 et les additifs inertes.

**12.** Procédé de lutte contre les parasites, caractérisé par le fait que l'on traite les parasites et/ou leur biotope avec une quantité efficace d'un 3-isoxazolylbenzyléther des formules générales Ia ou Ib selon la revendication 1.